Europäisches Patentamt

European Patent Office

Office européen des brevets

(11) Publication number: **0 403 976 B1**

# EUROPEAN PATENT SPECIFICATION

(45) Date of publication of patent specification: **25.01.95**

(51) Int. Cl.6: **C07C 5/41**, C07C 5/387, C10G 35/095

(21) Application number: **90111341.5**

(22) Date of filing: **15.06.90**

(54) **A method of producing aromatic hydrocarbons.**

(30) Priority: **19.06.89 JP 154484/90**

(43) Date of publication of application:
**27.12.90 Bulletin 90/52**

(45) Publication of the grant of the patent:
**25.01.95 Bulletin 95/04**

(84) Designated Contracting States:
**BE CH DE FR GB IT LI NL SE**

(56) References cited:
EP-A- 0 043 925
DE-A- 2 640 471
DE-B- 1 767 825
US-A- 4 644 089

(73) Proprietor: **RESEARCH ASSOCIATION FOR UTILIZATION OF LIGHT OIL**
**16-9, 2-chome, Shiba**
**Minato-ku**
**Tokyo (JP)**

(72) Inventor: **Yoneda, Toshikazu, C/o Central Laboratory**
**Idemitsu Kosan Co.,**
**1280 Kamiizumi Sodegauracho**
**Kimitsugun,**

Chiba prefecture (JP)
Inventor: **Imura, Kozo, C/o Kinuura laboratory**
**JGC Corporation,**
**2-110 Sunosakicho**
**Handa City,**
**Aichi Prefecture (JP)**
Inventor: **Inomata, Makoto, Kinuura laboratory**
**JGC Corporation,**
**2-110 Sunosakicho**
**Handa City,**
**Aichi Prefecture (JP)**
Inventor: **Nagamatsu, Shigeki, Kinuura laboratory**
**JGC Corporation,**
**2-110 Sunosakicho**
**Handa City,**
**Aichi Prefecture (JP)**

(74) Representative: **Reinhard, Skuhra, Weise**
**Postfach 44 01 51**
**D-80750 München (DE)**

**Description**

The present invention relates to a method of producing aromatic hydrocarbons from hydrocarbons containing $C_6$-$C_8$ paraffins, olefins or naphthenes.

DESCRIPTION OF THE PRIOR ART

As a highly active and selective aromatization catalyst, the Pt on L-type zeolite catalyst is well-known. For example, Japanese patent provisional publication Tokkaisho 61-283354 (1986) discloses a reforming catalyst of L-type zeolite containing metallic platinum and at least one additional metallic component as promoters to further the activity or selectivity of the basic platinum catalyst selected from a group consisting of iron, cobalt, titanium and rare earth metals, in which the mole ratio of platinum to additional metal is not larger than 10:1.

The reaction of producing aromatic hydrocarbons from paraffinic naphtha accompanies a so large endothermic quantity of heat (50-60 Kcal/mole) that it is required to supply the compensating heat of reaction.

As to the industrially established technology of producing aromatic hydrocarbons together with compensating the endothermic quantity of heat accompanied by the reaction, mentions are made for catalytic reforming reaction units producing aromatic hydrocarbons and gasoline fractions from $C_7$ (seven carbon atoms) or higher fractions on such processes as UOP(Platforming), CRC(Pheniforming), ERE-(Powerforming), Engelhard(Magnaforming), IFP(Aromizing), Standard Oil Indiana(Ultraforming), etc. All of these processes employ an adiabatic type multi-stage reactor in which catalysts are distributed in reactors in plural stages and heat is supplied between the reactors. In these cases, the temperature of feedstock is required to be unnecessarily heightened at the inlet of reactor in each stage.

Binary functional-type catalysts heretofore used for these catalytic reforming reaction units (Pt-Re-$Al_2O_3$-Cl, Pt-Ir-$Al_2O_3$-Cl, etc) are highly resistant to coking and exhibit effects stably under higher temperature reaction conditions. Accordingly, adiabatic type multi-stage reactors can be employed in which inlet temperatures of feedstock to be charged to the catalyst bed must be heightened to compensate the heat of the endothermic reaction.

The L-type zeolite catalyst carrying platinum used for the present invention is very sensitive to coking and is deactivated by the deposition of trace amounts of coke, though the catalyst is far superior in the aromatizing activity and selectivity in comparison with conventional binary functional-type catalysts. Therefore, the catalyst can not exhibit fully the superior characteristics when it is employed in conventionally used adiabatic type multi-stage reactors.

As to the catalytic reforming of petroleum fractions, Japanese patent publication Tokkosho 54-29528 (1979) proposes a method of supplying varying amounts of heat to the reaction mixture by providing successively into the tubular reactor a zone filled with inactive catalyst, a zone filled with diluted catalyst and a zone filled with non-diluted catalyst, and these zones are respectively equipped along them at outside of the reactor with a plural of separated electrical heating facilities or radiation heaters so as to supply heat to the reaction mixture passing successively through the above zones.

In this method, it is necessary to install electrical heating facilities or radiation heaters divided into plural numbers, and so a facility of complicated structure is requested. Further, the radiation heaters have structural difficulties when they are employed in an industrial scale.

SUMMARY OF THE INVENTION

It is an object of the present invention to provide a method of producing efficiently aromatic hydrocarbons from hydrocarbons containing $C_6$-$C_8$ paraffins, olefins or naphthenes.

BRIEF DESCRIPTION OF THE DRAWINGS

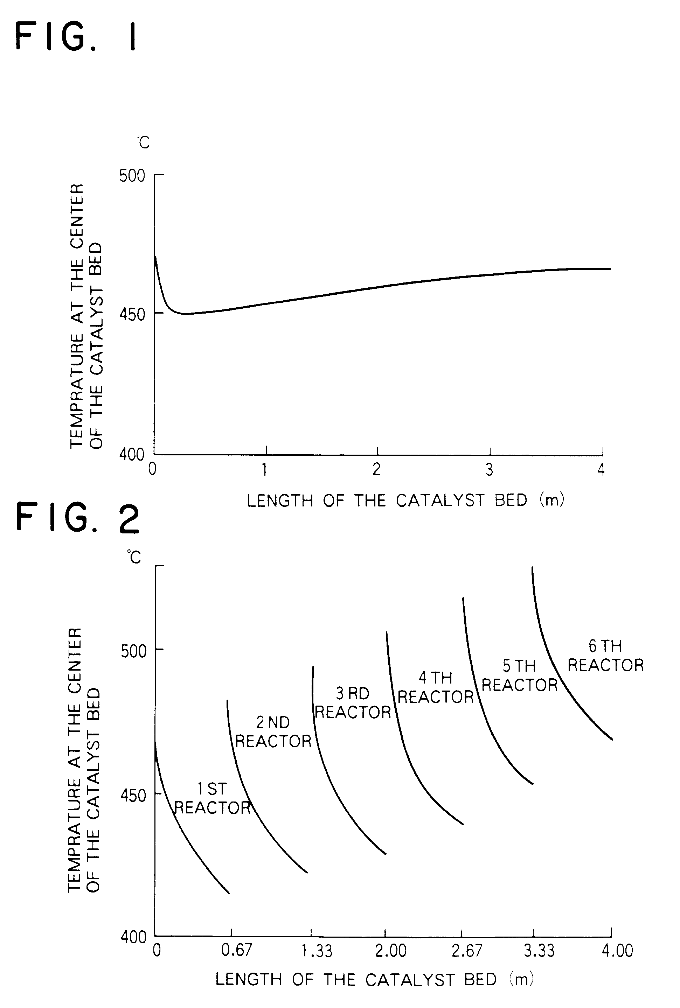

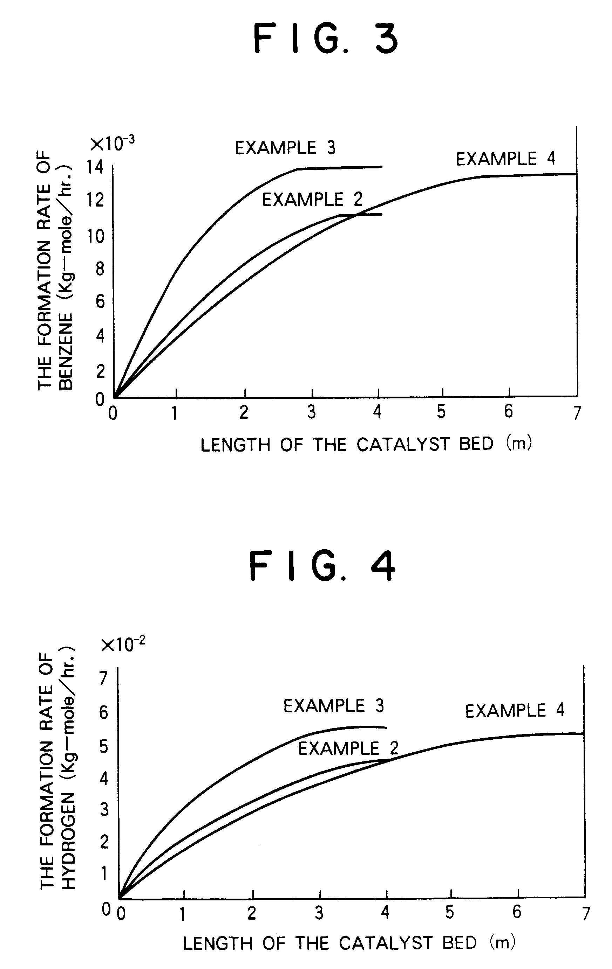

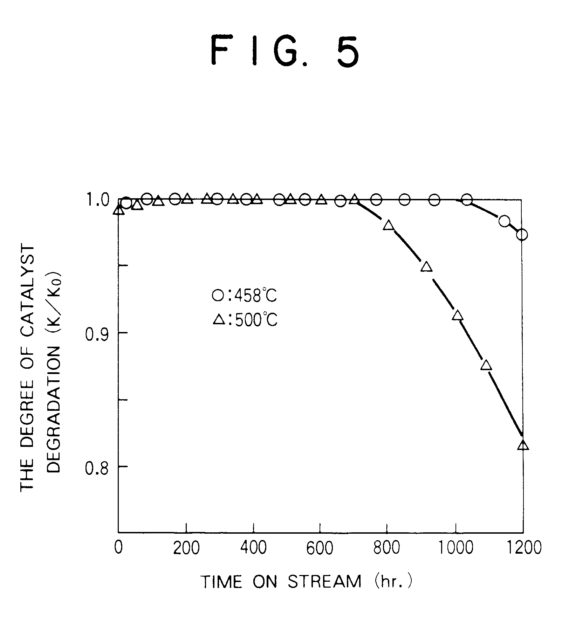

Fig.1 shows the temperature distribution in the externally heated tubular reactor of Example 1A, Fig.2 shows the temperature distribution in the adiabatic type multi-stage reactor of Comparative Example 1A, Fig.3 and Fig.4 show the results of Example 2-4 and Fig.5 shows the relationship between the time on stream and the catalyst degradation.

2

DETAILED DESCRIPTION OF THE PREFERRED EMBODIMENTS

In a method of producing aromatic hydrocarbons according to the present invention, L-type zeolite catalyst carrying platinum is filled in reaction tubes of an externally heated tubular reactor in which heat is provided to the reaction tubes externally through a heating medium, and a feedstock containing $C_6$-$C_8$ paraffins, $C_6$-$C_8$ olefins or $C_6$-$C_8$ naphthenes is charged into the reaction tubes to make contact with the catalyst in the presence of hydrogen.

The preferable amount of platinum carried on the L-type zeolite catalyst is in the range of 0.1-5.0 % by weight, more preferably in the range of 0.5-2.0 % by weight. As taught in Japanese patent provisional publication Tokkaisho 61-283354 (1986), platinum accompanied with an additional metallic component can be used.

As to the L-type zeolite, KL-type zeolite, especially FKL-zeolite obtained by treating KL-type zeolite with Flon gas, LiKL-zeolite or BaKL-zeolite treated with an alkali metal or alkaline earth metal for ion exchanging purpose are used preferably.

For the reactor, externally heated tubular reactors must be used. Preferable inner diameter of the tube is in the range of 15-50 mm (millimeter), especially in the range of 20-30 mm. When the diameter is smaller than 15 mm, number of the reaction tubes becomes too much and uneconomical, and the diameter larger than 50 mm makes the heat supply to be the rate-determining to result in low yields. Heating mediums for heating externally the reacting tubes are not restricted specifically, and such a medium as molten metal or molten salt may be used with merits of providing simplified and compact industrial units operable easily.

The preferable reaction temperature (temperature at the center of the catalyst bed) is in the range of 400-530°C, more preferably in the range of 430-500°C. When it is below 400°C, the yield of aromatics turns to low due to the equilibrium limitation of the dehydrogenation-cyclization reaction, and temperatures above 530°C proceeds the decomposition reaction and causes decrease in the activity.

The preferable reaction pressure is in the range of 1-15 atm. (1.01-15.2 bar), more preferably in the range of 2-10 atm (2.02-10.13 bar). Pressures below 1 atm. causes decrease in the activity due to coke deposition on catalyst, and pressures above 15 atm. lowers the yield of aromatics due to equilibrium limitation of the dehydrogenation-cyclization reaction.

Preferable mole ratio of $H_2$/HC (hydrocarbon) is in the range of 0.01-10, more preferably in the range of 0.25-6. When the ratio is below 0.01, the activity is decreased by coke deposition on catalyst, and the ratio above 10 results lower yields of aromatics due to the equilibrium limitation of the dehydrogenation-cyclization reaction and due to hydrogenolysis.

The preferable WHSV is in the range of 0.5-5/hr, more preferably in the range of 1-2/hr. When it is below 0.5/hr, the reactor capacity is requested to be larger, and above 5/hr provides insufficient activity.

The present invention will be explained concretely hereunder with examples. Examples 1-4 are results of calculation with a reforming simulator obtained by the analysis of reaction rate.

[Example 1A and Comparative Example 1A]

18.6 m³ of spherical catalyst particles (Catalyst A) having a diameter of 1.5 mm and carrying Pt on Flon gas-treated KL-type zeolite (Pt carried: 1.0 wt.%) were charged in an externally heated tubular reactor having a structure as shown in Table 1A, and an aromatization reaction (Example 1A) was conducted using light naphtha containing 84 % by weight of $C_6$ fraction and 16% by weight of $C_7$ fraction as the feedstock under reaction conditions as shown in Table 1A. The conversion and the selectivity of the reaction are shown in Table 1.

The same amount of the same catalyst as used in Example 1A were charged in an adiabatic type multi-stage (6 stages) reactor as shown in Table 1 and an aromatization reaction (Comparative Example 1A) was conducted using the same feedstock as used in Example 1A, regulating the temperature at feedstock inlet of reactor of each stage so as to obtain nearly the same conversion of the feedstock as that of Example 1A under the same operating conditions other than the temperatures at feedstock inlet of the reactors. The conversion and the selectivity of the reaction are shown in Table 1A.

The temperature distribution inside of the externally heated tubular reactor is shown in Fig.1, and the temperature distribution inside of the adiabatic type multi-stage reactor is shown in Fig. 2. In Fig.1 and Fig.2, the abscissa indicates the length (m: meter) of the catalyst bed, and the ordinate indicates temperatures (°C) at the center of the catalyst bed.

WHSV = Weight Hourly Space Velocity =

$$\frac{\text{Weight of hydrocarbons charged hourly}}{\text{Weight of catalyst in the catalyst Bed}}$$

Table 1A

| | Example 1A | Comparative Example 1A | | | | | |
|---|---|---|---|---|---|---|---|
| | Externally heated tubular reactor | Adiabatic type multi-stage reactor | | | | | |
| | | 1st | 2nd | 3rd | 4th | 5th | 6th |
| Outer diameter of tube (mm) | 27.2 | | | | | | |
| Thickness of tube (mm) | 2.5 | | | | | | |
| Inner diameter of tube(mm) | 22.2 | | | | | | |
| Number of tubes | 12000 | | | | | | |
| Length of catalyst bed (mm) | 4000 | 670 | 670 | 670 | 670 | 670 | 670 |
| Catalyst charged ($m^3$) | 18.6 | 3.1 | 3.1 | 3.1 | 3.1 | 3.1 | 3.1 |
| Temperature at feedstock inlet ($^\circ$C) | 475 | 470 | 482 | 494 | 506 | 518 | 530 |
| Inlet pressure (atm) | 8.0 | 8.0 | | | | | |
| Outlet pressure (atm) | 5.7 | 5.8 | | | | | |
| WHSV (1/HR) | 1.5 | 1.5 | | | | | |
| Feedstock hydrocarbon | Light naphtha | Light naphtha | | | | | |
| $H_2$/Hydrocarbon mole ratio | 5.0 | 5.0 | | | | | |
| Conversion (%) | 80.5 | 79.7 | | | | | |
| Selectivity (wt.%) | | | | | | | |
| $C_1$-$C_4$ | 4.9 | 5.9 | | | | | |
| $C_5$ | 3.3 | 3.6 | | | | | |
| Benzene | 69.8 | 69.0 | | | | | |
| Toluene | 14.1 | 13.7 | | | | | |
| Hydrogen | 7.9 | 7.8 | | | | | |

Comparing Fig.1 and Fig.2, it is apparent that the average temperature of the catalyst bed of the externally heated tubular reactor is lower than the average temperature of the catalyst bed of the adiabatic type reactor to result in lower forming ratios of $C_1$-$C_5$ decomposition products as shown in Table 1.

Since there is a tendency that the catalyst life of Pt carrying L-type zeolite is shortened in proportion to the amount of decomposition products (olefins), the externally heated tubular reactor producing less amount of the decomposition products can achieve a longer catalyst life.

[Example 1B and Comparative Example 1B]

With the same externally heated tubular reactor as used in Example 1A charged with Catalyst B [spherical particles having a diameter of 2.0 mm and carrying Pt on Flon gas-treated KL-type zeolite (Pt carried: 1.0 wt.%)], an aromatization reaction (Example 1B) was conducted using the same feedstock as used in Example 1A under operating conditions as shown in Table 1B. The conversion and selectivity of the reaction are shown in Table 1B.

With the same adiabatic type multi-stage reactor as used in Comparative Example 1A charged with the same catalyst as used in Example 1B, an aromatization reaction (Comparative Example 1B) was conducted using the same feedstock as used in Example 1A and operating conditions were controlled so as to have

4

EP 0 403 976 B1

the nearly equal average temperature of the catalyst bed as that of Example 1B. The conversion and selectivity of the reaction are shown in Table 1B.

Table 1B

| | Example 1B | Comparative Example 1B | | | | | |
|---|---|---|---|---|---|---|---|
| | Externally heated tubular reactor | Adiabatic type multi-stage reactor | | | | | |
| | | 1st | 2nd | 3rd | 4th | 5th | 6th |
| Outer diameter of tube (mm) | 27.2 | | | | | | |
| Thickness of tube (mm) | 2.5 | | | | | | |
| Inner diameter of tube(mm) | 22.2 | | | | | | |
| Number of tubes | 12000 | | | | | | |
| Length of catalyst bed (mm) | 4000 | 670 | 670 | 670 | 670 | 670 | 670 |
| Catalyst charged ($m^3$) | 18.6 | 3.1 | 3.1 | 3.1 | 3.1 | 3.1 | 3.1 |
| Temperature at feedstock inlet (°C) | 470 | 470 | 482 | 494 | 506 | 518 | 530 |
| Inlet pressure (atm) | 4.5 | 6.0 | | | | | |
| Outlet pressure (atm) | 2.6 | 3.7 | | | | | |
| WHSV (1/HR) | 1.5 | 1.5 | | | | | |
| Feedstock hydrocarbon | Light naphtha | Light naphtha | | | | | |
| $H_2$/Hydrocarbon mole ratio | 0.5 | 5.0 | | | | | |
| Conversion (%) | 90.9 | 77.6 | | | | | |
| Selectivity (wt.%) | | | | | | | |
| $C_1$-$C_4$ | 1.9 | 2.4 | | | | | |
| $C_5$ | 1.7 | 1.8 | | | | | |
| Benzene | 73.0 | 72.3 | | | | | |
| Toluene | 16.5 | 16.7 | | | | | |
| Hydrogen | 6.9 | 6.8 | | | | | |

The externally heated tubular reactor achieved a higher conversion and yield (yield = conversion x selectivity) of aromatic hydrocarbons in comparison with the adiabatic type reactor.

In the case of externally heated tubular reactor, the $H_2$/Hydrocarbon mole ratio was reduced to 0.5, because the heat of reaction was supplied from outside. On the contrary ,in the case of adiabatic type reactor, the $H_2$/Hydrocarbon mole ratio must be maintained at 5.0, because the heat of reaction must be supplied internally. If the $H_2$/Hydrocarbon mole ratio in the adiabatic type reactor was reduced to 0.5, the conversion and yield of aromatic hydrocarbons would fall further because of insufficient supply of the heat of reaction.

[Comparative Example 1C]

With the same adiabatic type multi-stage reactor as used in Comparative Example 1A charged with the same catalyst as used in Example 1B, an aromatization reaction (Comparative Example 1C) was conducted using the same feedstock as used in Example 1A regulating the temperature at feedstock inlet of reactor of each stage to 510°C. The conversion and selectivity of the reaction are shown in Table 1C together with the conversion and selectivity of Example 1B.

The externally heated tubular reactor achieved a higher conversion and yield (yield = conversion x selectivity) of aromatic hydrocarbons and lower forming ratios of $C_1$-$C_5$ decomposition products in comparison with the adiabatic type reactor.

5

EP 0 403 976 B1

Table 1C

| | Example 1B | Comparative Example 1C | | | | | |
|---|---|---|---|---|---|---|---|
| | Externally heated tubular reactor | Adiabatic type multi-stage reactor | | | | | |
| | | 1st | 2nd | 3rd | 4th | 5th | 6th |
| Outer diameter of tube (mm) | 27.2 | | | | | | |
| Thickness of tube (mm) | 2.5 | | | | | | |
| Inner diameter of tube(mm) | 22.2 | | | | | | |
| Number of tubes | 12000 | | | | | | |
| Length of catalyst bed (mm) | 4000 | 670 | 670 | 670 | 670 | 670 | 670 |
| Catalyst charged ($m^3$) | 18.6 | 3.1 | 3.1 | 3.1 | 3.1 | 3.1 | 3.1 |
| Temperature at feedstock inlet (°C) | 470 | 510 | 510 | 510 | 510 | 510 | 510 |
| Inlet pressure (atm) | 4.5 | 6.0 | | | | | |
| Outlet pressure (atm) | 2.6 | 3.6 | | | | | |
| WHSV (1/HR) | 1.5 | 1.5 | | | | | |
| Feedstock hydrocarbon | Light naphtha | Light naphtha | | | | | |
| $H_2$/Hydrocarbon mole ratio | 0.5 | 5.0 | | | | | |
| Conversion (%) | 90.9 | 81.2 | | | | | |
| Selectivity (wt.%) | | | | | | | |
| $C_1$-$C_4$ | 1.9 | 2.6 | | | | | |
| $C_5$ | 1.7 | 2.0 | | | | | |
| Benzene | 73.0 | 72.4 | | | | | |
| Toluene | 16.5 | 16.1 | | | | | |
| Hydrogen | 6.9 | 6.9 | | | | | |

[Example 2]

In an externally heated tubular reactor mentioned in Table 2 (the same with that of used in Example 1A) was filled with spherical Pt(1.0)/FKL-type zeolite catalyst having 1.5 mm diameter (the same with that of used in Example 1A). A mixture of $C_6$ fraction composed of i-$C_6$: 0.261 mole %, n-$C_6$: 0.576 mole % and methylcyclopentane: 0.163 mole % with hydrogen of 5 mole times of the $C_6$ fraction was charged at the rate of 1278 kg mole/hr with the feedstock inlet temperature of 470°C.

[Example 3]

A test was conducted under the same conditions as Example 2 except that the feedstock inlet temperature was 500°C.

[Example 4]

Using an externally heated tubular reactor mentioned in Table 2 (inner diameter of tube was 28.0 mm), a test was conducted under employment of the catalyst and feedstock as used in Example 2.

Dimensions of reactors and reaction conditions for Example 2-4 are shown in Table 2, and the test results are shown in Fig.3 and Fig.4.

6

Table 2

|  | Example 2 | Example 3 | Example 4 |
|---|---|---|---|
| Outer diameter of tube (mm) | 27.2 | 27.2 | 34.0 |
| Thickness of tube (mm) | 2.5 | 2.5 | 3.0 |
| Inner diameter of tube (mm) | 22.2 | 22.2 | 28.0 |
| Number of tubes | 12000 | 12000 | 6466 |
| Length of tube (mm) | 4000 | 4000 | 7000 |
| Catalyst charged ($m^3$) | 18.6 | 18.6 | 27.8 |
| Temperature at feedstock inlet (°C) | 470 | 500 | 500 |
| Inlet pressure (atm) | 6.30 | 6.30 | 7.30 |

In Fig.3, the abscissa indicates the length (m) of the catalyst bed, and the ordinate indicates the formation rate of benzene. In Fig.4. the abscissa indicates length of the catalyst bed (m), and the ordinate indicates the formation rate (kg. mole/hr) of hydrogen. The formation rate of benzene and formation rate of hydrogen are figures per one reaction tube.

Results of Example 2 and Example 3 are compared to notice that the feedstock inlet temperature of 470°C (Example 2) nearly bring about the equilibrium condition at 4 m length of the catalyst bed, while the feedstock inlet temperature of 500°C (Example 3) nearly brings about the equilibrium condition at 3 m length of the catalyst bed and the equilibrium value is higher.

Results of Example 3 and Example 4 are compared to notice that employment of the reactor having a larger inner diameter (28.0 mm) tube in Example 4 shows slowness in reaching the equilibrium condition and the equilibrium value is lower despite of the larger amount of catalyst charged, when compared with Example 3 employing the reactor having a smaller inner diameter (22.2 mm) tube.

[Example 5 and Example 6]

In a reaction tube having 20 mm inner diameter was charged 500g Pt(1.0) KL-type zeolite (16.32 mesh), and the tube was set into a fluidized bath heating furnace to subject to a reduction treatment of under $H_2$ gas flow, inlet pressure 6 atm., 540°C for 20 hrs. Then, reactions were carried out under the following conditions:

Feedstock : n-$C_6H_{14}$

$H_2$/feedstock mole ratio: 5

Inlet pressure: 6 atm.

WHSV : 2.0/hr

Feedstock inlet temperature;  Example 5: 500°C

Example 6: 458°C

The test results are shown in Table 3.

Table 3

| | Feedstock inlet temperature (°C) | Time on stream (hr) | Deposited carbon (wt.%) |
|---|---|---|---|
| Example 5 | 500 | 1200 | 0.23 |
| Example 6 | 458 | 1350 | 0.21 |

The amount of carbon deposited is smaller in one order when compared with conventional binary functional type catalysts.

Fig.5 shows the relationship between the time on stream and the degree of catalyst degradation. In Fig.5, the abscissa indicates the time on stream, and the ordinate indicates the degree of catalyst degradation defined by the following formula:

Degree of catalyst degradation = $K/K_0$

wherein

7

K = formation rate of aromatics at arbitrary time t
K₀ = formation rate of aromatics at initial stage of reaction

From Table 3 and Fig.5, it is recognized that, from the viewpoint of catalyst life, initiation of the reaction from at a temperature lower than 500°C is preferred.

**Claims**

1. In a method of producing aromatic hydrocarbons which comprises contacting a feedstock containg $C_6$ - $C_8$ paraffins, $C_6$ - $C_8$ olefins or $C_6$ - $C_8$ naphthenes with L-type zeolite catalyst carrying platinum in the presence of hydrogen, the improvement comprises the contact is undertaken in reaction tubes of an externally heated tubular reactor in which heat is provided to the reaction tubes externally through a heating medium.

2. A method of producing aromatic hydrocarbons according to claim 1, wherein the inner diameter of the tube is in the range of 15-50 millimeter, preferably in the range of 20-30 millimeter.

3. A method of producing aromatic hydrocarbons according to claim 1, wherein the heating medium is a molten metal or a molten salt.

4. A method of producing aromatic hydrocarbons according to claim 1, wherein the reaction temperature at the centre of the catalyst bed is in the range of 400 - 530°C, preferably in the range of 430 - 500°C.

5. A method of producing aromatic hydrocarbons according to claim 1, wherein the mole ratio of $H_2$ to hydrocarbon is in the range of 0.01-10, preferably in the range of 0.25-6.

6. A method of producing aromatic hydrocarbons according to claim 1, wherein the reaction pressure is in the range of 1-15 atm (1.01-15.2 bar), preferably in the range of 2-10 atm (2.02-10.13 bar).

7. A method of producing aromatic hydrocarbons according to claim 1, wherein the WHSV is in the range of 0.5-5/hr, preferably in the range of 1-2/hr.

**Patentansprüche**

1. Verfahren zur Herstellung aromatischer Kohlenwasserstoffe, umfassend das In-Kontakt-Bringen eines $C_6$-$C_8$-Paraffine, $C_6$-$C_8$-Olefine oder $C_6$-$C_8$-Naphthene enthaltenden Ausgangsmaterials mit einem platintragenden Zeolith-Katalysator vom L-Typ in Gegenwart von Wasserstoff, wobei die Verbesserung darin besteht, daß der Kontakt in Reaktionsröhren aus einem extern erhitzten röhrenförmigen Reaktor durchgeführt wird, indem die Hitze zu den Reaktionsröhren extern durch ein Erhitzungsmedium zugeführt wird.

2. Verfahren zur Herstellung aromatischer Kohlenwasserstoffe nach Anspruch 1, wobei der Innendurchmesser der Röhre im Bereich von 15-50 mm, bevorzugt im Bereich von 20-30 mm, liegt.

3. Verfahren zur Herstellung aromatischer Kohlenwaserstoffe nach Anspruch 1, wobei das Erhitzungsmedium eine Metallschmelze oder eine Salzschmelze ist.

4. Verfahren zur Herstellung aromatischer Kohlenwasserstoffe nach Anspruch 1, wobei die Reaktionstemperatur am Zentrum des Katalysatorbettes im Bereich von 400-530°C, bevorzugt im Bereich von 430-500°C, liegt.

5. Verfahren zur Herstellung aromatischer Kohlenwasserstoffe nach Anspruch 1, wobei das Mol-Verhältnis von $H_2$ zu Kohlenwasserstoff im Bereich von 0,01-10, bevorzugt im Bereich von 0,25-6, liegt.

6. Verfahren zur Herstellung aromatischer Kohlenwasserstoffe nach Anspruch 1, wobei der Reaktionsdruck im Bereich von 1-15 Atm (1,01-15,2 Bar), bevorzugt im Bereich 2-10 Atm (2,02-10,13 Bar), liegt.

7. Verfahren zur Herstellung aromatischer Kohlenwasserstoffe nach Anspruch 1, wobei der WHSV im Bereich von 0,5-5/h, bevorzugt im Bereich von 1-2/h, liegt.

**Revendications**

1. Dans un procédé de production d'hydrocarbures aromatiques qui consiste à mettre une matière première, contenant des paraffines en $C_6$-$C_8$, des oléfines en $C_6$-$C_8$ ou des naphtènes en $C_6$-$C_8$, en contact avec un catalyseur de zéolithe de type L portant du platine, en présence d'hydrogène, le perfectionnement consistant à effectuer le contact dans des tubes de réaction d'un appareil de réaction de type tubulaire à chauffage extérieur dans lequel de la chaleur est fournie extérieurement aux tubes de réaction par l'intermédiaire d'un agent chauffant.

2. Procédé de production d'hydrocarbures aromatiques selon la revendication 1, selon lequel le diamètre intérieur du tube est compris entre 15 et 50 millimètres, de préférence entre 20 et 30 millimètres.

3. Procédé de production d'hydrocarbures aromatiques selon la revendication 1, selon lequel l'agent chauffant est un métal fondu ou un sel fondu.

4. Procédé de production d'hydrocarbures aromatiques selon la revendication 1, selon lequel la température de réaction au centre du lit de catalyseur est comprise entre 400 et 530°C, de préférence entre 430 et 500°C;

5. Procédé de production d'hydrocarbures aromatiques selon la revendication 1, selon lequel le rapport molaire de $H_2$ à l'hydrocarbure est compris entre 0,01 et 10, de préférence entre 0,25 et 6.

6. Procédé de production d'hydrocarbures aromatiques selon la revendication 1, selon lequel la pression de réaction est comprise entre 1 et 15 atmosphères (entre 1,01 et 15,2 bars), de préférence entre 2 et 10 atmosphères (entre 2,02 et 10,13 bars).

7. Procédé de production d'hydrocarbures aromatiques selon la revendication 1, selon lequel le WHSV est compris entre 0,5 et 5 heures, de préférence entre 1 et 2 heures.

# FIG. I

°C

TEMPRATURE AT THE CENTER OF THE CATALYST BED

500

450

400

0      1      2      3      4

LENGTH OF THE CATALYST BED (m)

# FIG. 2

°C

TEMPRATURE AT THE CENTER OF THE CATALYST BED

500

450

400

1ST REACTOR

2ND REACTOR

3RD REACTOR

4TH REACTOR

5TH REACTOR

6TH REACTOR

0    0.67    1.33    2.00    2.67    3.33    4.00

LENGTH OF THE CATALYST BED (m)

# F I G. 3

THE FORMATION RATE OF BENZENE (Kg-mole/hr.)

×10⁻³

EXAMPLE 3
EXAMPLE 2
EXAMPLE 4

LENGTH OF THE CATALYST BED (m)

# F I G. 4

THE FORMATION RATE OF HYDROGEN (Kg-mole/hr.)

×10⁻²

EXAMPLE 3
EXAMPLE 2
EXAMPLE 4

LENGTH OF THE CATALYST BED (m)

# F I G. 5